# EUROPEAN PATENT SPECIFICATION

(11) **EP 2 697 152 B1**
(45) Date of publication and mention of the grant of the patent: **11.03.2015**
(21) Application number: 12717072.8
(22) Date of filing: 10.04.2012
(51) Int. Cl.: B67D 1/04, B67D 1/07, B67D 1/08, A61L 2/00, A61L 2/232

(54) **A METHOD OF PREVENTING BACTERIAL GROWTH IN A BEVERAGE DISPENSING SYSTEM**
VERFAHREN ZUR VORBEUGUNG VON BAKTERIENWACHSTUM IN EINER GETRÄNKESPENDEVORRICHTUNG
METHODE POUR LA PREVENTION DE LA PROLIFERATION BACTERIENNE DANS UN DISTRIBUTEUR DE BOISSONS

(30) Priority: 11.04.2011 EP 11161863
(43) Date of publication of application: 19.02.2014
(73) Proprietor: Carlsberg Breweries A/S, 1799 Copenhagen V (DK)
(72) Inventor: RASMUSSEN, Jan Nørager, 3650 Ølstykke (DK); VESBORG, Steen, 2820 Gentofte (DK)
(74) Representative: Budde Schou A/S
(86) International application number: PCT/EP2012/056413
(87) International publication number: WO 2012/139995

(56) References cited:
- EP-A1- 1 216 953
- EP-A1- 1 489 048
- US-A1- 2008 203 113

## Description

The present invention relates to a method of preventing bacterial growth in a beverage dispensing system, a beverage container for preventing bacterial growth in a beverage dispensing system, a beverage dispensing assembly for preventing bacterial growth comprising a beverage dispensing system and a beverage container, and a container connector for a beverage container for preventing bacterial growth.

### BACKGROUND

Beverage dispensing systems are typically used in beverage dispensing establishments for efficiently dispensing large quantities of beverage. Typically, beverage dispensing systems are used to dispense carbonated alcoholic beverages such as draught beer and cider. However, also non-alcoholic beverages such as soft drinks and non-carbonated beverages such as wine and fruit juice may be dispensed using a beverage dispensing system Beverage dispensing systems are typically intended for professional establishments like bars, restaurants and hotels, however, increasingly also for private users such as in private homes.

In the present context, keg and container are considered to be synonymous words. Beverage dispensing systems for providing draught beverages from exchangeable beverage containers are well known in the art. One example from the prior art is US 3,889,487 disclosing a system in which a beverage keg is connected by a flexible conduit to a faucet on a faucet standard. The keg is kept in a refrigerated chamber and the beverage is pressurized by supplying carbon dioxide into the keg. The beverage is dispensed from the faucet.

When the beverage keg is empty, it is removed and returned to the beverage producer Since most beverages include nutrients and other substances which may promote bacterial growth it is important to clean and sterilize the kegs before re-filling them with beverage. Insufficient cleaning may lead to unhygienic beverage kegs, which may in turn lead to bacterial growth causing health problems for the beverage consumer.

As an alternative to the above rigid beverage kegs, due to the above hygiene concern, beverage kegs are increasingly made collapsible and for single use only. Such kegs use different beverage dispensing systems. Examples of such beverage dispensing systems include systems produced by the applicant company under the name DraughtMaster^{™} and described in the applicant's international patent applications WO 2007/019848, WO 2007/019849, WO 2007/019850, WO 2007/019851, WO 2007/ 019852. In these kinds of beverage dispensing systems, the beverage container is placed inside a pressure chamber. Beverage is forced out of the keg into a dispensing line by using an external gas pressure to collapse the container.

While performing a dispensing operation, the force of the pressure causes the beverage to flow out of the beverage container and into a tapping line. The tapping line, typically constituted by a flexible tube, leads to a tapping cock typically having a dispensing valve and a tapping handle for allowing an operator to control the beverage dispensing operation. The operator, such as a bartender or barmaid, uses the tapping handle to control the rate of beverage dispensing. After each beverage dispensing operation, residual beverage will inevitably be left in the tapping line and in the tapping cock. On the opposite side of the tapping line a tapping line connector provides fluid communication between the beverage container and the tapping line. The tapping line connector is also known as a tapping head or keg coupler.

After a certain amount of time a layer of residual beverage may be formed inside the tapping line and tapping device. Such layers of residual beverage may solidify and eventually clog the tapping line and/or the tapping cock, which will impair the beverage dispensing operation. However, well before clogging the tapping line and/or tapping device, the residual beverage will pose a hygienic problem. The tapping line and the tapping device constitute areas where bacterial growth may be accelerated due to the presence of beverage, the large surface area in comparison to the beverage volume, the lack of sufficient cooling and the close proximity to the outside Bacterial growth due to lack of hygiene in the tapping line and the tapping device may constitute a quality problem for the beverage consumer. Additionally, crust formation of solidified beverage within the tapping line may occur.

The above problems have been solved by frequent cleaning of the dispensing line and tapping device. Methods and systems for cleaning and flushing the dispensing line are disclosed in the applicant's international applications WO 2010/060949, WO 2010/ 060946 and WO 2010/029122. In these applications, a cleaning fluid is caused to enter the dispensing line and be dispensed at the dispensing device. The cleaning fluid removes any residual beverage or contamination remaining in the tapping line or tapping cock. Subsequently, the dispensing line is flushed by water to remove the remaining cleaning fluid.

The applicant has noted that in the existing systems the connection between the tapping line and the beverage container is not cleaned and thus constitutes a location in which microorganisms (microbes) and similar contamination may enter the beverage container and/or the dispensing line. The connection between the tapping line and the container is typically established each time a new container is installed by connecting a tapping line connector to a container connector. The container connector is then exchanged together with the exchanging of the beverage container and may thus be considered to be essentially sterile, at least when being protected by a suitable lid, whereas the tapping line connector is exchanged significantly less frequently, if ever exchanged, and thus microorganisms and contamination may accumulate on and within the tapping line connector. When the beverage container is exchanged, such microorganisms may enter the tapping line and thus contaminate the beverage. There is thus a need for technologies for preventing bacterial growth at the tapping line connector prior to or simultaneously with connecting it to a new container connector. This need has been addressed in the prior art, as can be seen below.

EP 2 164 794 relates to a gasket for mounting on a beverage container. The gasket is permanently deformed when the beverage container is removed from a fluid passage such that the user has to provide a new gasket each time the beverage container is exchanged thereby preventing microbial growth However, the user still has to perform the manual step of exchanging the gasket, and in case no additional gaskets are available, beverage dispensing will be interrupted.

EP 1 216 953 relates to a tapping head for drawing beverages under gas pressure. The tapping head has a channel for feeding a disinfecting agent to the area of the beverage closing valve for disinfecting the fitting area.

US 7 237 566 relates to a device for cleaning and/or disinfecting a keg coupler. An adapter can be fitted onto the keg coupler The adapter comprises an applying unit adapted to apply a cleaning liquid onto the keg coupler

US 2009/0249566 relates to a container top cleaner. The cleaner is used to wipe the lids of cans, bottles and other containers prior to use.

The drawback of the above solutions is the fact that the cleaning agent must be provided separately each time a new container is provided. However, the user may forget to use the cleaning agent every time a new container is provided or the use of cleaning agent may even be omitted deliberately for economical and time saving reasons.

The object of the present invention is thus to achieve a prevention of bacterial growth on the tapping line connector without having to use any manual intervention.

Other prior art includes WO 2008/089909 relating to a beverage pouring device in the form of a disposable barrel. The filling and pouring of the device is made by means of a safety fitting which consists of a sterile material. However, there is no advice at all that the tapping line connector or the tapping line is sterile.

US 2007/0137726 relates to a beverage dispenser with an outlet for dispensing beverages. An ultraviolet light source is directed at the outlet for disinfecting the outlet. However, there is no advice that the tapping line connector or the tapping line is thereby disinfected. It is thus an advantage according to the present invention that the tapping line connector is disinfected.

A beverage container according to the preamble of claim 12 is known from EP-A-1 216 953.

### SUMMARY OF THE INVENTION

The above need, the above advantage and the above object together with numerous other needs, advantages and objects, which will be evident from the below detailed description, are according to a first aspect of the present invention obtained by a method of preventing bacterial growth in a beverage dispensing system, the method comprising the steps of:
providing the beverage dispensing system comprising a tapping cock and a tapping line connected at its first end to the tapping cock and having at its opposite second end a tapping line connector, the tapping line connector having a tapping valve actuator operable between a beverage dispensing position and a non-beverage dispensing position and defining a first connector surface,
providing a beverage container filled with a beverage preferably being a carbonated beverage, the beverage container having a container connector defining a second connector surface and an initially sealed container opening, the second connector surface being covered with a bacteriostatic agent, the bacteriostatic agent being in solid state or gel state, the container connector being connectable to the tapping line connector by contacting the first and second connector surfaces with one another,
connecting the tapping line connector to the container connector and in doing so transferring a part of the bacteriostatic agent from the second connector surface to the first connector surface by contacting the first and second connector surfaces with one another, and
operating the tapping valve actuator from the non-beverage dispensing position to the beverage dispensing position and in doing so establishing fluid communication from the interior of the beverage container via the opening and the tapping line to the tapping cock.

The tapping cock typically constitutes a faucet for allowing the user to selectively dispense beverage as long as any beverage is remaining in the container. The tapping cock is typically connected to a tapping rod (faucet standard) at an elevated position in relation to a bar counter. The container is typically located in a refrigerator or cold storage room, either just below the bar counter or in a separate room such as in a cellar or basement or the like. The tapping line leading from the container to the tapping cock is typically a flexible conduit, however, it may also, at least partially, be in the form of a metal tubing permanently installed in the beverage dispensing establishment Thus, the tapping line and the tapping cock are typically not replaced each time the beverage container is exchanged, while the beverage container is replaced with a new one each time it is exhausted. The tapping line and the tapping cock should be cleaned as often as necessary in order to ensure a hygienic dispensing. The beverage container typically contains carbonated beverage such as beer.

The tapping line has a tapping line connector located at an opposite end relative to the tapping cock. The tapping line connector is connectable to a corresponding container connector on the beverage container. The connection may include e.g. a screw connection, a bayonet connection or a press fit connection. The tapping line connector comprises a first surface which, upon connection, contacts a second surface of the container connector. The container connector includes an opening for allowing beverage to leave the container via the container connector. The tapping line connector includes a tapping valve actuator for controlling the flow of beverage through the tapping line. The tapping valve actuator is part of a tapping valve and may act on a closing member located in the tapping line connector, a closing member located in the container connector, or both. The closing member closes off the container opening and/or the tapping line when the tapping valve actuator is held in the non-beverage dispensing position, and, establishes fluid communication through the container opening when the tapping valve actuator is held in the beverage dispensing position. Thus, only when both the tapping valve actuator and the dispensing valve are opened, beverage may flow from the beverage container to the tapping cock. The tapping line connector may also include a check valve for preventing any residual beverage stored within the tapping line from escaping in a reverse direction. Further, the tapping valve may in some embodiments be used for closing off the tapping line connector during rinsing of the tapping line for avoiding rinsing fluid or cleaning fluid entering the beverage container. Thus, the rinsing fluid will not be able to reach the parts of the tapping line connector and the tapping valve located outside of the location where the tapping valve closing member closes the tapping line and/or container opening.

During connection of the tapping line connector onto the container connector, the first surface and the second surface will contact. The first surface encompasses most or all of the inner surfaces of the tapping line connector and in particular the parts of the tapping line connector which is exposed to beverage. Preferably, also the outwardly oriented parts of the tapping valve are encompassed by the first surface. The second surface thereby encompasses the corresponding surface on the beverage container connector which will contact the first surface.

The expression bacteriostatic agent is understood to include chemical substances and mixtures which prevent the growth of microorganisms such as bacteria and fungi. Since the second surface is covered with a bacteriostatic agent, the contacting of the first and second surfaces will result in transfer of a part of the bacteriostatic agent from the second surface to the first surface. Since the bacteriostatic agent is provided at the second surface, each exchange of the container will result in an automatic transfer of bacteriostatic agent to the first surface of the tapping line connector. Thus, by providing the bacteriostatic agent as a cover at the second surface of the container connector already at the beverage producer, it will not be possible for a user to forget or omit the prevention of bacterial growth at the tapping line connector, since it is performed automatically upon installation of the beverage container. Further, the kind and amount of bacteriostatic agent may be pre-determined at the supplier or beverage producer in an automated process and thus there is no or little risk of over-dosing or under-dosing the amount of the bacteriostatic agent at the location of the user when exchanging the beverage container.

The bacteriostatic agent may preferably be a paste or gel of bacteriostat, fungistat or a similar preservative substance which retains the growth rate of any microorganisms present on the first and second surfaces. Such microorganisms located on the first surface of the tapping line connector may otherwise quickly reproduce to a large number which may enter the beverage during exchange of beverage container. The bacteriostatic agent prevents such reproduction and thus prevents that a large number of microorganisms develop near the beverage container opening. The bacteriostatic agent may further be disinfecting such that the amount of microorganisms present on the first and second surface is reduced. Preventing bacterial growth should in the present context be construed broadly to encompass all kinds of suppressing of bacterial and microbiological reproduction.

The bacteriostatic agent may also comprise a coating of the second surface of the container connector such that when the container connector is connected to the tapping line connector, the coated second surface rubs onto the first surface and thereby prevents bacterial growth on the first surface.

Although in the past wooden kegs were common for storing draught beverage, most draught beverage is today provided in metal (steel) containers or plastic (PET) containers Both metal containers and plastic containers are exchangeable, i.e. the user is provided with a full beverage container from the beverage producer, e.g. the brewery. The full container is connected to the beverage dispensing system of the user. In case of a metal container the empty container is removed and returned to the producer for cleaning and refilling. In the case of a plastic container it is disposed of by combustion after it has been exhausted. The beverage dispensing system will differ slightly depending on whether it is used together with plastic or metal containers. During dispensing from plastics containers, a pressurized gas is typically applied outside the container in order to provide a beverage driving pressure and ensure that the container is collapsing while the beverage is ejected. During dispensing from metal containers, the beverage driving pressure is achieved by pressurized gas being applied within the container and the container will thus retain its shape during dispensing. Thus, the tapping line connector and consequently also the container connector for a metal container beverage dispensing system will include, apart from the opening allowing beverage to leave the container, a further opening for allowing pressurized gas to enter the container. Alternatively, a single large opening is used.. The tapping line connector and consequently also the container connector for a plastic container beverage dispensing system will include only the opening for allowing beverage to leave the container.

After the tapping lie connector is connected to the container connector and the tapping valve actuator is operated from the non-beverage dispensing position to the beverage dispensing position, fluid communication is established between the beverage container and the tapping line via the opening of the container connector. The first and second surfaces thereby also establish the fluid communication and seal the tapping line connector and the container connector with respect to the outside.

According to a further embodiment of the method, the second connector surface is circumferentially encircling the container opening. In order to achieve a complete disinfection of the tapping line connector, the second connector surface may encircle the opening in order to not allow any part of the tapping line connector to remain contaminated.

According to a further embodiment of the method, the container connector constitutes a male connector and the tapping line connector constitutes a female connector, or vice versa. In order to achieve both a secure and fluid tight connection and a proper disinfection of the tapping line connection, the tapping line connector and the container connector may be constituted by inter-connectable male and female connectors, respectively In some embodiments, the container connector may be of female type and thus enclosing the tapping line connector being of male type. In other embodiments, the tapping line connector may be of female type and thus enclosing the container connector being of male type.

According to a further embodiment of the method, the bacteriostatic agent is at least partially covering the opening. The bacteriostatic agent may cover the opening in order to contact other parts of the first surface which are not contacted by the second surface. Those parts may be parts which may be exposed to beverage, e.g. outwardly oriented parts of the tapping valve such as a valve piston which may be exposed to beverage and contamination.

According to a further embodiment of the method, the bacteriostatic agent comprises a cleaning tablet located within the container connector and contacting the second surface. The bacteriostatic agent may be in the form of a tablet which is loosely positioned within the container connector such that when the container connector is connected to the tapping line connector, the tablet is crushed and distributed in-between the first and second surfaces and consequently the bacterial growth at the first surface is prevented.

According to a further embodiment of the method, the bacteriostatic agent is water soluble. By providing a water soluble bacteriostatic agent, the bacteriostatic agent will dissolve when being contacted by beverage. The initial beverage amount which enters the interior of the container connector and tapping line connector after the beverage container has been pressurized and/or the tapping valve has been opened may thus dissolve some or all of the bacteriostatic agent. The resulting beverage/bacteriostatic agent mixture may reach and prevent bacterial growth at parts of the tapping line connector which have possibly not been in contact with the second surface and received a direct prevention of bacterial growth. In this way it is ensured that the whole interior of the tapping line connector and possibly also the whole interior of the tapping valve will be substantially free from bacteria or other microorganisms.

According to a further embodiment of the method, the bacteriostatic agent is non-toxic. Since it is inevitable that some bacteriostatic agent will enter the beverage, the bacteriostatic agent should be non-toxic. In order for the bacteriostatic agent to not influence the taste of the beverage, it also preferably is tasteless. In this way any small amount of bacteriostatic agent which may enter the beverage does not harm the health of the user or influences the taste of the beverage if it is accidentally consumed. In practice, a very small amount of dissolved bacteriostatic agent will be part of the beverage which is dispensed after the changing of the container. The anti-bacterial agent should thus not have a pH very different from the beverage in order to not provoke excessive foaming of the beverage to be served.

According to a further embodiment of the method, the bacteriostatic agent comprises one or more of potassium sorbate, sodium benzonate, citric acid, tartaric acid, hydrophilic silica, 1,2 dihydroxy propane and glycerol. Citric acid is a bacteriostatic agent which is considered to be non-toxic for the present purposes and which is suitable for use in the container connector. Preferably, the following recipe is used:

| | |
|---|---|
| Potassiumsorbate | 0.1 % |
| Sodiumbenzoate | 0.1 % |
| Citric acid or tartaric acid | 0.3 % |
| Hydrophilic silica (Aerosil) | 10 % |
| 1,2 dihydroxy propane | 0 to 30 % |
| Glycerol | 99.8 % |

The viscosity of glycerol is strongly dependent on water content Hydrophobic silica is used as a thickener. 1.2 dihydroxy propane (nontoxic propyleneglycol) is used for controlling overall "viscosity" and to suppress crystallization of glycerol. In order to prepare the anti-microbal agent, all of the ingredients except citric acid is heated under stirring to 70 degrees centigrade. Subsequently, the heating is cut off, and citric acid is added and everything stirred till room temperature. After deaeration it is ready to be dispensed as a paste or gel. In order to apply the mixture as a gel, it may also include up to 20% of PEG 4000 or PEG 6000. The mixture may be applied as a hot (about 55 degrees centigrade), melted gel, which later solidifies. Preferably a vacuum mixing apparatus is used for making the mixture. Such apparatus are e.g. provided by the companies Petzhoit, Dobb or Werner & Pfleiderer. The gel may be applied using a hot glue gun.

According to a further embodiment of the method, the bacteriostatic agent has a water activity (A_{w}) of less than 0.7. Such low levels of water activity will support only a very low amount of microorganisms, thus having a bacteriostatic effect.

According to a further embodiment of the method, the container opening is initially closed off by a ball valve operable by the tapping valve actuator. In order to prevent any contamination from entering the beverage within the container and prevent any beverage from spilling to the outside, the container opening may be closed off by a ball valve. The ball valve may be spring-loaded for securely sealing and to allow an automatic re-sealing of the beverage container in case the tapping line connector is removed from the container connector The ball valve may be pushed from closed position to open position when the tapping valve actuator is moved from the non-beverage dispensing position to the beverage dispensing position.

According to a further embodiment of the method, the container opening is initially covered by a piercable membrane, the piercable membrane being pierced during the connecting of the tapping line connector to the container connector. In addition to or instead of a ball valve, to prevent any contamination to enter the beverage container during transport and handling, the opening of the beverage container may be provided with a piercable membrane, e.g. a thin plastic or metal sheet covering the opening. When the tapping line connector is applied onto the container connector, the membrane may be pierced for allowing fluid communication between the interior of the beverage container and the tapping line connector. In addition to the above, a removable cap of rigid plastic or metal may cover the membrane in order to prevent accidental piercing of the membrane.

According to a further embodiment of the method, the beverage container has a volume of between 5-50 litres, preferably between 10-25 litres, such as 10-15 litres, 15-20 litres or 20-25 litres. The above volumes constitute typical volumes of exchangeable beverage kegs. Such kegs thus having a size which can be easily handled by a user while allowing multiple servings of beverage without the need of changing beverage container.

The above need, the above advantage and the above object together with numerous other needs, advantages and objects, which will be evident from the below detailed description, are according to a second aspect of the present invention obtained by a beverage container for a beverage dispensing system, the beverage dispensing system comprising a tapping cock and a tapping line connected at its first end to the tapping cock and having at its opposite second end a tapping line connector, the tapping line connector having a tapping valve actuator operable between a beverage dispensing position and a non-beverage dispensing position and defining a first connector surface, the beverage container being filled by a beverage preferably being a carbonated beverage, the beverage container having a container connector defining a second connector surface and an initially sealed container opening, the second connector surface being covered with a bacteriostatic agent, the bacteriostatic agent being in solid state or gel state, the container connector being connectable to the tapping line connector by contacting the first and second connector surfaces with one another and in doing so transferring a part of the bacteriostatic agent from the second connector surface to the first connector surface. The above beverage container according to the second aspect is preferably used together with the above method of sterilizing a beverage dispensing system according to the first aspect.

The above need, the above advantage and the above object together with numerous other needs, advantages and objects, which will be evident from the below detailed description, are according to a third aspect of the present invention obtained by a beverage dispensing assembly comprising a beverage dispensing system and a beverage container, the beverage dispensing system comprising a tapping cock and a tapping line connected at its first end to the tapping cock and having at its opposite second end a tapping line connector, the tapping line connector having a tapping valve actuator operable between a beverage dispensing position and a non-beverage dispensing position and defining a first connector surface, the beverage container being filled by a beverage preferably being a carbonated beverage, the beverage container having a container connector defining a second connector surface and an initially sealed container opening, the second connector surface being covered with a bacteriostatic agent, the bacteriostatic agent being in solid state or gel state, the container connector being connectable to the tapping line connector by contacting the first and second connector surfaces with one another and in doing so transferring a part of the bacteriostatic agent from the second connector surface to the first connector surface. The above beverage dispensing assembly according to the third aspect is preferably used together with the above method of sterilizing a beverage dispensing system according to the first aspect and/or the above beverage container according to the second aspect.

The above need, the above advantage and the above object together with numerous other needs, advantages and objects, which will be evident from the below detailed description, are according to a fourth aspect of the present invention obtained by a container connector for a beverage container for a beverage dispensing system, the beverage dispensing system comprising a tapping cock and a tapping line connected at its first end to the tapping cock and having at its opposite second end a tapping line connector, the tapping line connector having a tapping valve actuator operable between a beverage dispensing position and a non-beverage dispensing position and defining a first connector surface, the beverage container being filled by a beverage preferably being a carbonated beverage, the container connector being adapted to be mounted at a mouth of a beverage container for defining an initially sealed container opening, the container connector defining a second connector surface, the second connector surface being covered with a water soluble bacteriostatic agent, the bacteriostatic agent being in solid state or gel state, the container connector being connectable to the tapping line connector by contacting the first and second connector surfaces with one another and in doing so transferring a part of the bacteriostatic agent from the second connector surface to the first connector surface. The container connector may be provided as a separate part which is connected to a state of the art metal or plastic container by the user. The above container connector according to the fourth aspect is preferably used together with the above method of sterilizing a beverage dispensing system according to the first aspect, the above beverage container according to the second aspect and/or the above beverage dispensing assembly according to the third aspect.

### BRIEF DESCRIPTION OF THE FIGURES

Fig 1 is a cut-out view of a plastic container and a corresponding container connector.
Fig 2 is a cut-out view of a metal container and a corresponding container connector.

### DETAILED DESCRIPTION OF THE FIGURES

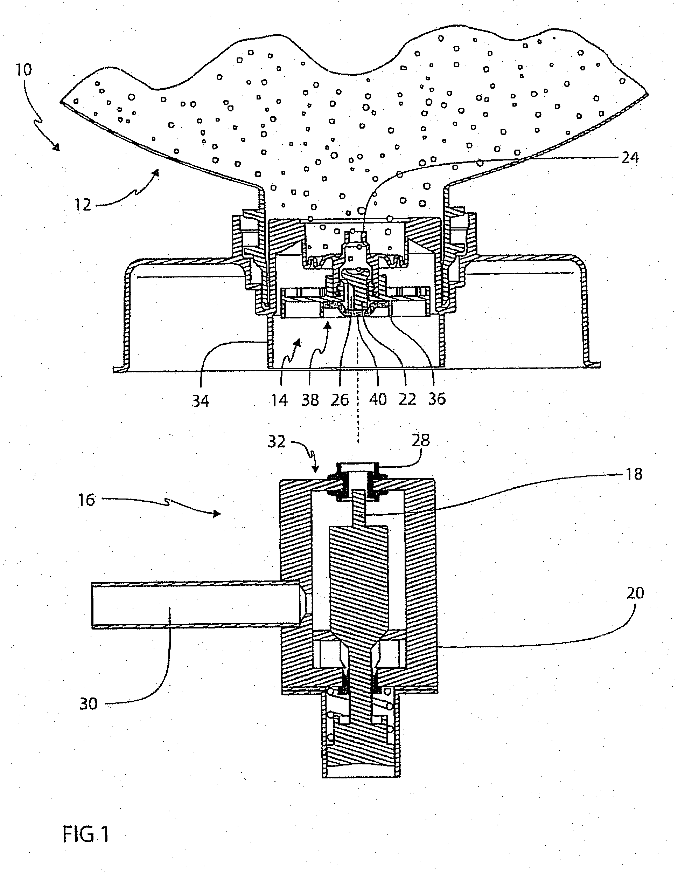

Fig 1 shows a schematic and vertical sectional view of a beverage dispensing assembly 10 comprising a beverage container 12 being made by PET or a similar plastics and having a body part (only partially shown) being filled by beverage. The beverage container 12 further comprises a beverage container connector 14 which is connected to a neck part of the beverage container 12. The beverage dispensing assembly 10 further includes a tapping line connector 16 onto which the container connector 14 is connectable. During beverage dispensing, the beverage container 12 and the tapping line connector 16 are both positioned within a pressure space (not shown) which is pressurised by a pressure fluid for forcing the beverage out of the beverage container 12 into the tapping line connector 16 by collapsing the beverage container 12.

The tapping line connector 16 comprises a tapping valve actuator 18, which is located inside a hollow housing 20. The tapping valve actuator 18 is adapted to act on a closure element 22. The closure element 22 and the tapping valve actuator together constitute a tapping valve. The closure element 22 is in the present embodiment not a part of the tapping line connector, but part of the beverage container 12. The closure element 20 is received in the container connector 14. The present tapping valve actuator 18 is operable between three possible positions, which constitute a first position, and opposite second position and an intermediate position. As will be described in greater detail below, the intermediate position constitutes a beverage dispensing position, the first positions constitute a closed position and the second position constitutes an optional rinsing position. The first and second positions both constitute non beverage dispensing positions.

The closure element 22 is in the present embodiment located in the container connector in a specific space between a constriction 24 and an opening 26. When the beverage container 12 is mounted on the tapping line connector 16, beverage may flow from the beverage container 12 through a gasket 28 of the housing 20, via the interior of the housing 20 towards a tapping line 30. The tapping line 30 leads to a tapping cock (not shown) located on a tapping rod (not shown) of a bar counter (not shown), all of which is generally known in the prior art.

Both the inlet constriction 24 and the opening constitute valve seats, which the closure element 22 may seal against The closure element 22 will initially establish a seal against the opening 26. The opening 26 is established by the container connector 14 which serves to fixate the container connector 14 relative to a first surface 32 of the housing 20 of the tapping line connector 16. The container connector is provided with an outer circumferential rim 34 extending downwardly and a further interior downwardly extending circumferential flange 36 both serving to position the container connector 14, and consequently the beverage container 12, in a specific upright position relative to the tapping line connector 16.

The tapping line connector 16 may be loose in relation to the pressure chamber (not shown), however, it is typically fixated to the bottom wall of the pressure chamber (not shown), i.e. the tapping line connector 16 including the housing 20 and the valve actuator 18 remain with the bottom wall of the pressure chamber (not shown). The valve actuator 18 and the housing 20 may thus be made of rigid and non-disposable materials such as metal, particularly stainless steel or aluminium. When the beverage container 12 is installed onto the tapping line connector 16, the beverage container 12 is swung or pivoted into an upside down horizontal orientation such that the first surface 32 of the housing 20 is positioned as indicated in fig 1 in a juxtaposed position in relation to a second connector surface 38 located between the opening 26 and the rim 34 of the container connector 14. Upon connecting the container connector 14 and the tapping line connector, the first connector surface 32 and the second connector surface 38 will contact and fluid tight communication between the opening 26 and the gasket 28 will be established. When the beverage container 12 together with the container connector 14 is removed from the tapping line connector 16, the closure element 22 remains with the container connector 14 of the beverage container 12.

Initially, when a new sealed beverage container 12 is installed, the container connector 14 is sealed off by a laminate covering or a lid. The closure element 22 is further positioned in the first closed position and seals the opening of the container connector 14. The closure element 22 may optionally be spring-loaded towards the first closed position and/or the opening may include means for keeping the closure element 22 in the first closed position for preventing that beverage leaks from the container during shipping and handling. When the container connector 14 is installed onto the tapping line connector 16, the valve actuator 18 initially is assuming the first closed position, i.e., at a distant position in relation to the beverage container 12 and the opening 26 thus remains closed.

When the valve actuator 28 is in the beverage dispensing position, i.e. in the active or intermediate position, the closure element 22 is located in an intermediate position between the inlet constriction 24 and the opening 26 as the bottom end of the closure element 22 is resting on the top of the valve actuator and at the same time the valve actuator 18 is disengaged from contact with the gasket 28 allowing free passage from the inlet constriction 24 passed the closure element 22 via the opening 26 and the gasket 28 and further into the tapping line 30 via the housing 20.

From the beverage dispensing position the valve actuator 18 may be shifted further towards the beverage container 12 to a second rinsing position in which the valve actuator 18 seals against gasket 28 and simultaneously the top of the valve actuator 18 is pushing the closure element into a sealing contact with the inlet constriction 24. The present embodiment constitutes a particular safe configuration since in the rinsing position, the interior of beverage container 12 will be sealed off twice in relation to the housing 20, i.e. both at the gasket 28 and at the inlet constriction 24. Although such dual sealing is not mandatory, it will allow the interior of the housing 20 and the tapping line 30 to be rinsed by a rinsing fluid which may be unsuitable for human consumption and subsequently flushed by water without risking that any of the nasty rinsing fluid will enter the beverage in case one of the sealing locations' malfunction.

Upon removing the beverage container 12, the valve actuator 18 must be in the first closed position and the beverage, indicated by the signature of "circles" in the figure, will exert a force on the closure element 22 pushing the closure element 22 against the opening 26 defining the closed position, i.e. the second passive position, thereby sealing off the beverage container 12.

In the present context, the upper parts of the gasket 28 and the first connector surface 32 will not be rinsed or flushed and thus microbes such as bacteria and fungus may accumulate there. Some amounts of beverage may leak from the container onto those surfaces which may form locations for microbial growth Such microbes may migrate into the beverage container 12 and tapping line 30 and thus contaminate the beverage. To avoid this, the second surface 38 of the container connector 14 may be covered with a bacteriostatic agent in the form of a bacteriostastic paste or gel 40. When the container connector 14 is connected to the tapping line connector 16, the bacteriostatic paste 40 on the second connector surface 38 is rubbed onto the first connector surface 32 of the tapping line connector 16 and thus some of the bacteriostatic paste 40 will be transferred to the first connector surface 32 and the gasket 28. The first connector surface 32 and the gasket 28 will thus each time the beverage container is exchanged be subjected to the bacteriostatic paste 40 which will inhibit any bacterial growth on the first connector surface 32 and the gasket 28 in-between the exchange of beverage containers 12. The bacteriostatic paste 40 may optionally also cover the opening 26 and the closure element 22 in order to achieve a proper prevention of bacterial growth on the top of the valve actuator 18. The present assembly is fool proof in that the prevention of bacterial growth is automatic upon installation of a new beverage container and cannot be forgotten or deliberately omitted by the user. The bacteriostatic paste preferably constitutes a thin layer of between a few hundred micrometers up to a few millimetres thickness and being solid or semisolid such that it remains in place during handling but smears onto the first surface during contact with the second surface. The baceriostatic paste should preferably be non-toxic and tasteless since it may be transferred to the beverage in small amounts.

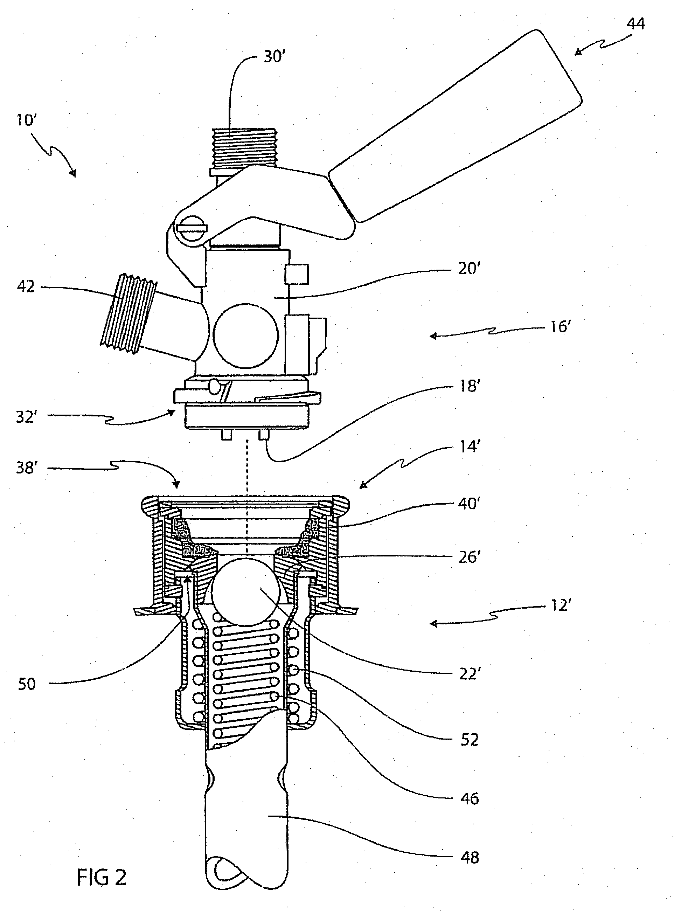

Fig 2 shows a schematic and vertical sectional view of a beverage dispensing assembly 10' similar to the assembly presented in connection with fig 1. The assembly 10' comprises a beverage container 12' being made by steel or a similar metal and having a body part (only partially shown) being filled by beverage. The beverage container 12' further comprises a beverage container connector 14' which is connected to a neck part of the beverage container 12'. The beverage dispensing assembly 10' further includes a tapping line connector 16' onto which the container connector 14' is connectable. During beverage dispensing, the beverage container 12' is pressurised by a pressure fluid which is fed into the beverage container 12' for forcing the beverage out of the beverage container 12' into the tapping line connector 16'.

The tapping line connector comprises a hollow housing 20'. The hollow housing 20' comprises a tapping line connector 30' connected to a tapping line and a pressure fluid connector 42 connected to a source of pressurized fluid. The hollow housing further comprises a valve actuator 18' which is operable between a beverage dispensing position and a non-beverage dispensing position by means of a handle 44.

The tapping line connector 16' is provided with a first connector surface 32' constituting a screw thread which allows access into the housing 20 and which is compatible with a corresponding screw thread screw located on a second connector surface 38' located on a second connector surface of the container connector 14'. The second connector surface is surrounding an opening 26' which allows access into the beverage container 12'.

Typically, the beverage container 12' is positioned in an upright position and the tapping line connector 16' is freely movable. When the tapping line connector 16 is connected to the container connector 14', the valve actuator 18' should be in a non-beverage dispensing position. In the non-beverage dispensing position a closure element 22' seals against the opening 26' of the beverage container 12'. The closure element is spring-loaded against the opening 26 by means of an inner spring 46 When the handle 44 is moved to the beverage dispensing position, the valve actuator 18' moves towards the closure element 26' and pushes the closure element 26' downwardly against the force of the inner spring 46 such that fluid communication is established from an ascending pipe 48 within the beverage container 12' passed the closure element 22' and via the opening 26' and the housing 20' to the tapping line connector 30'.

In addition to the above, pressure fluid, typically CO₂, is supplied into the pressure inlet 42 and led via a separate path into the beverage container 12' to a location outside the ascending pipe 48. The flow of pressure fluid in the housing 20' is completely separated from the flow of beverage in a coaxial relationship. The pressure fluid is led Into the beverage container 12' via a spring-loaded valve 50 established between the ascending pipe 48 and a valve seat 50. The ascending pipe 48 is thereby spring-loaded by an outer spring 52 at the opening 26' against the valve seat 50. When the pressure in the head space of the beverage container 12' sinks e.g. due to beverage dispensing, the pressure on the ascending pipe 48 will increase and the ascending pipe will temporary move downwardly thereby establishing fluid communication between the pressure inlet 42 and the beverage container 12'.

A bacteriostatic paste 40' is applied onto the second connector surface 38'. When the tapping line connector 16' is connected to the container connector 14' by engaging the screw threads of the first connector surface 32 and the second connector surface 38', some of the bacteriostatic paste 40' of the second connector surface 38' is transferred onto the first connector surface 32' of the tapping line connector 16' similar to the embodiment shown in fig 1. Thereby, the growth of bacteria and other microorganisms is prevented at the tapping line connector 16'. The recipe for the bacteriostatic paste is preferably:

| | |
|---|---|
| Potassiumsorbate | 0.1 % |
| Sodiumbenzoate | 0.1 % |
| Citric acid or tartaric acid | 03 % |
| Hydrophilic silica (Aerosil) | 10 % |
| 1,2 dihydroxy propane | 0 to 30 % |
| Glycerol | 99.8 % |

In order to prepare the anti-microbial agent, all of the ingredients except citric acid are heated under stirring to 70 degrees centigrade. Subsequently, the heating is cut off, and citric acid is added and everything stirred till room temperature. After deaeration it is ready to be dispensed as a paste or gel. In order to apply the mixture as a gel, it may also include up to 20% of PEG 4000 or PEG 6000. The mixture may be applied as a hot (about 55 degrees centigrade), melted gel, which later solidifies. Preferably a vacuum mixing apparatus is used for making the mixture. Such apparatus are e.g. provided by the companies Petzholt. Dobb or Werner & Pfleiderer. The gel may be applied using a hot glue gun.

Although the present invention has been described above with the reference to specific embodiment of the container and also of the connector, it is of course to be contemplated that numerous modifications be deduced by a person having ordinary skill in the art. For example, it may be contemplated to use a plastic container as shown in connection with fig 1 which is not to be collapsed but which is adapted to receive pressurised carbon dioxide gas within the container, similar to the metal container shown in fig 2.

### LIST OF PARTS WITH REFERENCE TO THE FIGURES

10. Beverage dispensing assembly
12. Beverage container
14. Container connector
16. Tapping line connector
18. Valve actuator
20. Housing
22. Closure element
24. Inlet constriction
26. Opening
28. Gasket
30. Tapping line
32. First connector surface
34. Rim
36. Flange
38. Second connector surface
40. Bacteriostatic paste
42. Pressure fluid inlet
44. handle
46. Inner spring
48. Ascending pipe
50. Spring-loaded valve
52. Outer spring

## Claims

1. A method of preventing bacterial growth in a beverage dispensing system, said method comprising the steps of:
providing said beverage dispensing system comprising a tapping cock and a tapping line (30) connected at its first end to said tapping cock and having at its opposite second end a tapping line connector (16), said tapping line connector (16) having a tapping valve actuator (18) operable between a beverage dispensing position and a non-beverage dispensing position and defining a first connector surface (32),
providing a beverage container (12) filled with a beverage preferably being a carbonated beverage, said beverage container (12) having a container connector (14) defining a second connector surface (38) and an initially sealed container opening (26), said second connector surface (38) being covered with a bacteriostatic agent, said bacteriostatic agent being in solid state or gel state, said container connector (14) being connectable to said tapping line connector (16) by contacting said first (32) and second (38) connector surfaces with one another,
connecting said tapping line connector (16) to said container connector (14) and in doing so transferring a part of said bacteriostatic agent from said second connector surface (38) to said first connector surface (32) by contacting said first (32) and second (38) connector surfaces with one another, and
operating said tapping valve actuator (18) from said non-beverage dispensing position to said beverage dispensing position and in doing so establishing fluid communication from the interior of said beverage container (12) via said opening and said tapping line (30) to said tapping cock.

2. The method according to claim 1, wherein said second connector surface (38) is circumferentially encircling said container opening (26).

3. The method according to any of the preceding claims, wherein said container connector (14) constitutes a male connector and said tapping line connector (16) constitutes a female connector, or vice versa:

4. The method according to any of the preceding claims, wherein said bacteriostatic agent is at least partially covering said opening (26).

5. The method according to any of the preceding claims, wherein said bacteriostatic agent comprises a cleaning tablet located within said container connector and contacting said second surface.

6. The method according to any of the claims 1-4, wherein said bacteriostatic agent is water soluble.

7. The method according to any of the preceding claims, wherein said bacteriostatic agent is non-toxic.

8. The method according to claim 7, wherein said bacteriostatic agent comprises one or more of potassium sorbate, sodium benzonate, citric acid, tartaric acid, hydrophilic silica, 1,2 dihydroxy propane and glycerol.

9. The method according to any of the preceding claims, wherein said bacteriostatic agent has a water activity (A_{w}) of less than 0,7.

10. The method according to any of the preceding claims, wherein said container opening is initially closed off by a ball valve operable by said tapping valve actuator.

11. The method according to any of the preceding claims, wherein said opening initially being covered by a piercable membrane, said piercable membrane being pierced during said connecting of said tapping line connector to said container connector.

12. A beverage container for a beverage dispensing system, said beverage dispensing system comprising a tapping cock and a tapping line connected at its first end to said tapping cock and having at its opposite second end a tapping line connector, said tapping line connector having a tapping valve actuator operable between a beverage dispensing position and a non-beverage dispensing position and dispensing position and defining a first connector surface (32), said beverage container (12) being filled by a beverage preferably being a carbonated beverage, **characterized in that** said beverage container (12) having a container connector (14) defining a second connector surface (38) and an initially sealed container opening (26), said second connector surface (38) being covered with a bacteriostatic agent, said bacteriostatic agent being in solid state or gel state, said container connector (14) being connectable to said tapping line connector (16) by contacting said first (32) and second (38) connector surfaces with one another and in doing so transferring a part of said bacteriostatic agent from said second connector surface (38) to said first connector surface (32).

13. A beverage dispensing assembly comprising a beverage dispensing system and a beverage container (12) according to claim 12; said beverage dispensing system comprising a tapping cock and a tapping line (30) connected at its first end to said tapping cock and having at its opposite second end a tapping line connector (16), said tapping line connector (16) having a tapping valve actuator (18) operable between a beverage dispensing position and a non-beverage dispensing position and defining a first connector surface (32).

14. A container connector (14) for a beverage container (12) according to claim 12, said beverage container (12) being adapted for use with a beverage dispensing system, said beverage dispensing system comprising a tapping cock and a tapping line (30) connected at its first end to said tapping cock and having at its opposite second end a tapping line connector (16), said tapping line connector (16) having a tapping valve actuator (18) operable between a beverage dispensing position and a non-beverage dispensing position and defining a first connector surface (32), said beverage container (12) being filled by a beverage, said container connector (14) being adapted to be mounted at a mouth of a beverage container (12) for defining an initially sealed container opening (26), said container connector (14) defining a second connector surface (38), said second connector surface (38) being covered with a water soluble bacteriostatic agent, said bacteriostatic agent being in solid state or gel state, said container connector (14) being connectable to said tapping line connector (16) by contacting said first (32) and second (38) connector surfaces with one another and in doing so transferring a part of said bacteriostatic agent from said second connector surface (38) to said first connector surface (32).

15. A method, beverage dispensing assembly, beverage container or container connector according to any of the preceding claims, wherein said beverage container having a volume of between 5-50 litres.

## Patentansprüche

1. Verfahren zur Vorbeugung von Bakterienwachstum in einer Getränkespendevorrichtung, wobei das Verfahren die folgenden Schritte umfasst:
Bereitstellen der Getränkespendevorrichtung umfassend einen Zapfhahn und eine Zapfleitung (30), die an ihrem ersten Ende mit dem Zapfhahn verbunden ist und an ihrem gegenüberliegenden zweiten Ende einen Zapfleitungsanschluss (16) aufweist, wobei der Zapfleitungsanschluss (16) einen Zapfventilaktuator (18) aufweist, der zwischen einer Getränkeabgabeposition und einer nicht-Getränkeabgabeposition betätigt werden kann und eine erste Anschlussfläche (32) definiert,
Bereitstellen eines Getränkebehälters (12), der mit einem Getränk gefüllt ist, das bevorzugt ein kohlensäurehaltiges Getränk ist, wobei der Getränkebehälter (12) einen Behälteranschluss (14) aufweist, der einen zweiten Anschlussfläche (38) und eine anfänglich verschlossene Behälteröffnung (26) definiert, wobei die zweite Anschlussfläche (38) mit einem bakteriostatischen Mittel bedeckt ist, wobei das bakteriostatische Mittel in einem festen Zustand oder Gelzustand vorliegt, wobei der Behälteranschluss (14) durch in-Berührung-bringen der ersten (32) und zweiten (38) Anschlussflächen miteinander mit dem Zapfleitungsanschluss (16) verbunden werden kann,
Verbinden des Zapfleitungsanschlusses (16) mit dem Behälteranschluss (14) und dadurch Übertragen eines Teils des bakteriostatischen Mittels von der zweiten Anschlussfläche (38) auf die erste Anschlussfläche (32) durch in-Berührung-bringen der ersten (32) und zweiten (38) Anschlussflächen miteinander, und
Betätigen des Zapfventilaktuators (18) von der nicht-Getränkeabgabeposition zu der Getränkeabgabeposition und dadurch Errichten von Fluidverbindung von dem Inneren des Getränkebehälters (12) über die Öffnung und die Zapfleitung zu dem Zapfhahn.

2. Verfahren nach Anspruch 1, wobei die zweite Anschlussfläche (38) die Behälteröffnung (26) umfangsmäßig umrandet.

3. Verfahren nach einem der vorherstehenden Ansprüche, wobei der Behälteranschluss (14) einen Verbindungsstecker bildet, und der Zapfleitungsanschluss (16) eine Buchse bildet, oder umgekehrt.

4. Verfahren nach einem der vorherstehenden Ansprüche, wobei das bakteriostatische Mittel wenigstens teilweise die Öffnung (26) bedeckt.

5. Verfahren nach einem der vorherstehenden Ansprüche, wobei das bakteriostatische Mittel eine Reinigungstablette umfasst, die innerhalb des Behälteranschlusses angeordnet ist und die zweite Fläche berührt.

6. Verfahren nach einem der Ansprüche 1-4, wobei das bakteriostatische Mittel wasserlöslich ist.

7. Verfahren nach einem der vorherstehenden Ansprüche, wobei das bakteriostatische Mittel nicht-toxisch ist.

8. Verfahren nach Anspruch 7, wobei das bakteriostatische Mittel ein oder mehrere von Kaliumsorbat, Natriumbenzonat, Zitronensäure, Weinsäure, hydrophobes Kieselsäure, 1,2 Dihydroxypropan und Glycerol umfasst.

9. Verfahren nach einem der vorherstehenden Ansprüche, wobei das bakteriostatische Mittel eine Wasseraktivität (A_{w}) von weniger als 0,7 aufweist.

10. Verfahren nach einem der vorherstehenden Ansprüche, wobei die Behälteröffnung anfänglich durch ein Kugelventil verschlossen ist, das durch den Zapfventilaktuator betätigt werden kann.

11. Verfahren nach einem der vorherstehenden Ansprüche, wobei die Öffnung anfänglich durch eine durchlochbare Membrane bedeckt ist, wobei die durchlochbare Membrane während der Verbindung des Zapfleitungsanschlusses mit dem Behälteranschluss durchlocht wird.

12. Getränkebehälter für eine Getränkespendevorrichtung, wobei die Getränkespendevorrichtung einen Zapfhahn und eine Zapfleitung umfasst, die an ihrem ersten Ende mit dem Zapfhahn verbunden ist und an ihrem gegenüberliegenden zweiten Ende einen Zapfleitungsanschluss aufweist, wobei der Zapfleitungsanschluss einen Zapfventilaktuator aufweist, der zwischen einer Getränkeabgabeposition und einer nicht-Getränkeabgabeposition und Abgabeposition betätigt werden kann und eine erste Anschlussfläche definiert, wobei der Getränkebehälter (12) mit einem Getränke gefüllt ist, das bevorzugt ein kohlensäurehaltiges Getränke ist, **dadurch gekennzeichnet, dass** der Getränkebehälter (12) einen Behälteranschluss (14) aufweist, der eine zweite Anschlussfläche (38) und eine anfänglich verschlossene Behälteröffnung (26) definiert, wobei die zweite Anschlussfläche (38) mit einem bakteriostatischen Mittel bedeckt ist, wobei das bakteriostatische Mittel in einem festen Zustand oder Gelzustand vorliegt, wobei der Behälteranschluss (14) durch in-Berührung-bringen der ersten und zweiten Anschlussflächen miteinander mit dem Zapfleitungsanschluss (16) verbunden werden kann und dadurch einen Teil des bakteriostatischen Mittels von der zweiten Anschlussfläche (38) auf die erste Anschlussfläche (32) überträgt.

13. Getränkeabgabeanordnung umfassend eine Getränkespendevorrichtung und einen Getränkebehälter (12) nach Anspruch 12, wobei die Getränkespendevorrichtung einen Zapfhahn und eine Zapfleitung (30) umfasst, die an ihrem ersten Ende mit dem Zapfhahn verbunden ist und an ihrem gegenüberliegenden zweiten Ende einen Zapfleitungsanschluss (16) aufweist, wobei der Zapfleitungsanschluss einen Zapfventilaktuator (18) aufweist, der zwischen einer Getränkeabgabeposition und einer nicht-Getränkeabgabeposition betätigt werden kann, und einen ersten Anschlussfläche (32) definiert.

14. Behälterverbindungselement (14) für einen Getränkebehälter (12) nach Anspruch 12, wobei der Getränkebehälter (12) zur Verwendung in einer Getränkespendevorrichtung vorgesehen ist, wobei die Getränkespendevorrichtung einen Zapfhahn und eine Zapfleitung (30) umfasst, die an ihrem ersten Ende mit dem Zapfhahn verbunden ist und an ihrem gegenüberliegenden zweiten Ende einen Zapfleitungsanschluss (16) aufweist, wobei der Zapfleitungsanschluss (16) einen Zapfventilaktuator (18) aufweist, der zwischen einer Getränkeabgabeposition und einer nicht-Getränkeabgabeposition betätigt werden kann und eine erste Anschlussfläche definiert, wobei der Getränkebehälter mit einem Getränke gefüllt ist, wobei der Behälteranschluss ausgestaltet ist, auf einer Mündung eines Getränkebehälters befestigt zu werden, um eine anfänglich verschlossene Behälteröffnung zu definieren, wobei der Behälteranschluss (14) eine zweite Anschlussfläche (38) definiert, wobei die zweite Anschlussfläche (38) mit einem wasserlöslichen bakteriostatischen Mittel bedeckt ist, wobei das bakteriostatische Mittel in einem festen Zustand oder Gelzustand vorliegt, wobei der Behälteranschluss (14) durch in-Berührung-bringen der ersten und zweiten Anschlussflächen miteinander mit dem Zapfleitungsanschluss (16) verbunden werden kann und dadurch einen Teil des bakteriostatischen Mittels von der zweiten Anschlussfläche (38) auf die erste Anschlussfläche (32) überträgt.

15. Verfahren, Getränkeabgabeanordnung, Getränkebehälter oder Behälterverbindungselement nach einem der vorherstehenden Ansprüche, wobei der Getränkebehälter ein Volumen von zwischen 5-50 Liter aufweist.

## Revendications

1. Méthode pour la prévention de la prolifération bactérienne dans un distributeur de boissons, ladite méthode comprenant les étapes de:
fournir ledit distributeur de boissons comprenant un robinet de distribution et un tuyau de distribution (30) raccordés à sa première extrémité audit robinet de distribution et ayant à sa deuxième extrémité opposée un raccordement de tuyau de distribution (16), ledit raccordement de tuyau de distribution (16) ayant un actionneur de vanne de distribution (18) opérable entre une position de distribution de boissons et une position de non-distribution de boissons et définissant une première surface de raccordement (32),
fournir un récipient pour boissons (12) rempli avec une boisson étant préférablement une boisson gazeuse, ledit récipient pour boissons (12) ayant un raccordement du récipient (14) définissant une deuxième surface de raccordement (38) et une ouverture de récipient initialement étanchéifiée (26), ladite deuxième surface de raccordement (38) étant couverte d'un agent bactériostatique, ledit agent bactériostatique étant dans un état solide ou un état gélifié, ledit raccordement du récipient (14) pouvant être raccordé audit raccordement de tuyau de distribution (16) en contactant lesdites première (32) et deuxième (38) surfaces de raccordement l'une à autre,
raccorder ledit raccordement de tuyau de distribution (16) audit raccordement du récipient (14) et en faisant ceci, transférer une partie de l'agent bactériostatique de ladite deuxième surface de raccordement (38) à ladite première surface de raccordement (32) en contactant lesdites première (32) et deuxième (38) surfaces de raccordement l'une à l'autre, et
opérer ledit actionneur de vanne de distribution (18) de ladite position de non-distribution de boissons à ladite position de distribution de boissons et en faisant ceci, établir de la communication fluide de l'intérieur dudit récipient pour boissons (12) par ladite ouverture et ledit tuyau de distribution (30) audit robinet de distribution.

2. Méthode selon la revendication 1, dans laquelle ladite deuxième surface de raccordement (38) encercle de manière circonférentielle ladite ouverture du récipient (26).

3. Méthode selon l'une quelconque des revendications précédentes, dans laquelle ledit raccordement du récipient (14) constitue une prise mâle ou vice versa.

4. Méthode selon l'une quelconque des revendications précédentes, dans laquelle ledit agent bactériostatique couvre au moins partiellement ladite ouverture (26).

5. Méthode selon l'une quelconque des revendications précédentes, dans laquelle ledit agent bactériostatique comprend une pastille de nettoyage localisée à l'intérieur dudit raccordement du récipient et contactant ladite deuxième surface.

6. Méthode selon l'une des revendications 1 à 4, dans laquelle ledit agent bactériostatique est soluble à l'eau.

7. Méthode selon l'une quelconque des revendications précédentes, dans laquelle ledit agent bactériostatique est non-toxique.

8. Méthode selon la revendication 7, dans laquelle ledit agent bactériostatique comprend un(e) ou plusieurs parmi les suivant(e)s: Sorbate de potassium, benzoate de sodium, acide citrique, acide tartrique, silice hydrophile, 1,2 dihydroxy-propane et glycérol.

9. Méthode selon l'une quelconque des revendications précédentes, dans laquelle ledit agent bactériostatique a une activité d'eau (A_{w}) inférieure à 0,7.

10. Méthode selon l'une quelconque des revendications précédentes, dans laquelle ladite ouverture de récipient est initialement fermée par une soupape à bille opérable par l'actionneur de ladite vanne de distribution.

11. Méthode selon l'une quelconque des revendications précédentes, dans laquelle ladite ouverture étant initialement couverte par une membrane brisable, ladite membrane brisable étant brisée pendant ledit raccordement dudit raccordement de tuyau de distribution audit raccordement du récipient.

12. Récipient pour boissons pour un distributeur de boissons, ledit distributeur de boissons comprenant un robinet de distribution et un tuyau de distribution raccordés à sa première extrémité audit robinet de distribution et ayant à sa deuxième extrémité opposée un raccordement de tuyau de distribution (16), ledit raccordement de tuyau de distribution (16) ayant un actionneur de vanne de distribution (18) opérable entre une position de distribution de boissons et une position de non-distribution de boissons et définissant une première surface de raccordement (32), ledit récipient pour boissons (12) étant rempli par une boisson étant préférablement une boisson gazeuse, **caractérisé en ce que** le récipient pour boissons (12) a un raccordement du récipient (14) définissant une deuxième surface de raccordement (38) et une ouverture de récipient initialement étanchéifiée (26), ladite deuxième surface de raccordement (38) étant couverte d'un agent bactériostatique, ledit agent bactériostatique étant en état solide ou en état gélifié, ledit raccordement du récipient (14) pouvant être raccordé audit raccordement de tuyau de distribution (16) en contactant lesdites première (32) et deuxième (38) surfaces de raccordement l'une à l'autre et en faisant ceci, transférer une partie dudit agent bactériostatique de ladite deuxième surface de raccordement (38) à ladite première surface de raccordement (32).

13. Assemblage de distribution de boissons comprenant un distributeur de boissons et un récipient pour boissons (12) selon la revendication 12, ledit distributeur de boissons comprenant un robinet de distribution et un tuyau de distribution (30) raccordés à sa première extrémité audit robinet de distribution et ayant à sa deuxième extrémité opposée un raccordement de tuyau de distribution (16), ledit raccordement de tuyau de distribution (16) ayant un actionneur de vanne de distribution (18) opérable entre une position de distribution de boissons et une position de non-distribution de boissons et définissant une première surface de raccordement (32).

14. Raccordement du récipient (14) pour un distributeur pour boissons (12) selon la revendication 12, ledit récipient pour boissons (12) étant adapté pour utilisation avec un distributeur de boissons, ledit distributeur de boissons comprenant un robinet de distribution et un tuyau de distribution (30) raccordés à sa première extrémité audit robinet de distribution et ayant à sa deuxième extrémité opposée un raccordement de tuyau de distribution (16), ledit raccordement de tuyau de distribution (16) ayant un actionneur de vanne de distribution (18) opérable entre une position de distribution de boissons et une position de non-distribution de boissons et définissant une première surface de raccordement (32), ledit récipient pour boissons (12) étant rempli avec une boisson, ledit raccordement du récipient (14) étant adapté à être monté à une bouche d'un récipient pour boissons (12) pour définir une ouverture de récipient initialement étanchéifiée (26), ledit raccordement du récipient (14) définissant une deuxième surface de raccordement (38), ladite deuxième surface de raccordement (38) étant couverte d'un agent bactériostatique soluble à l'eau, ledit agent bactériostatique étant dans un état solide ou un état gélifié, ledit raccordement du récipient (14) étant raccordé audit raccordement de tuyau de distribution (16) en contactant lesdites première (32) et deuxième (38) surfaces de raccordement l'une à l'autre et en faisant ceci, transférer une partie de l'agent bactériostatique de ladite deuxième surface de raccordement (38) à ladite première surface de raccordement (32).

15. Méthode, assemblage de distribution de boissons, récipient pour boissons ou raccordement du récipient selon l'une quelconque des revendications précédentes, dans lesquels ledit récipient pour boissons a un volume entre 5 et 50 litres.
